# EUROPEAN PATENT SPECIFICATION

(11) **EP 2 167 052 B1**
(45) Date of publication and mention of the grant of the patent: **01.04.2015**
(21) Application number: 08762672.7
(22) Date of filing: 17.06.2008
(51) Int. Cl.: A61K 9/50, A61K 9/16, A61F 13/20, A61K 33/18

(54) **BIOCIDE CAPSULES, PREPARATION COMPRISING THEM AND SANITARY PAD COMPRISING THE PREPARATION**
BIOZID-KAPSELN, DIESE ENTHALTENDE ZUBEREITUNG UND DIE ZUBEREITUNG UMFASSENDE HYGIENEBINDE
CAPSULES BIOCIDES, PRÉPARATION LES CONTENANT ET TAMPON SANITAIRE COMPRENANT LA PRÉPARATION

(30) Priority: 18.06.2007 HU 0700418; 12.06.2008 HU 0800375
(43) Date of publication of application: 31.03.2010
(73) Proprietor: Dolhay Klinika Egészségügyi KFT., 1037 Budapest (HU)
(72) Inventor: DOLHAY, Balázs, H-1037 Budapest (HU)
(74) Representative: Derzsi, Katalin
(86) International application number: PCT/HU2008/000069
(87) International publication number: WO 2008/155589

(56) References cited:
- WO-A-95/15157
- US-A- 4 582 052
- US-A- 4 623 588
- US-A- 5 070 889
- US-B1- 6 328 991
- PARK, HEE-MYUNG ET AL: "Production and characterization of biodegradable povidone iodine microsphere as a intramammary disinfectant" JOURNAL OF VETENARY INTERNAL MEDICINE, vol. 64, no. 8, 2002, pages 739-741, XP008104137

## Description

The invention is directed to biocide capsules, preparation comprising the biocide capsules and tampon comprising the preparation.

The biocide active ingredient is released from the capsule of the invention in the presence of pathogens, the capsule of the invention, or the preparations contain at least one biocide active ingredient.

The preparation is used for healthcare purposes, primarily for killing the pathogens of the body surface, body orifices, open and closed body cavities. The capsule of the invention, particularly the preparation can advantageously be used for the treatment and/or diagnosis of the vagina.

According to the invention the biocide active ingredients are the bactericide, viricide, fungicide, insecticide, etc. substances known for one skilled in the art.

It is known that the vaginal epithelium and Döderlein flora of the sexually mature woman maintains a protecting acid sheet with pH=3,5-4,3. In abnormal conditions discharge is dripping from the true pelvis, oviduct, uterus, cervix into the vagina, and this is less acidic. Substances getting into the vagina from the outside are also significantly less acidic. Discharges of the inflammation or tumour developing on the portio vaginalis uteri and on the vaginal wall are also less acidic. As a result of what is mentioned above, the pH of the whole vagina or a limited part of it shifts into the neutral or even alkaline region. Usually this is the way as the acidic sheet generated by the irregularly layered epithelial cells, covering the multilayered, not keratinizing epithelium, and the normal flora ceases to exit. This is why that instead of the Döderlein flora corresponding to the normal pH, facultative or obligate pathogens proliferate, in the pH environment suitable for them. Those pathogens colonize which erode or totally dissolve the layered epithelium and the interstitial substance, and even the connective tissue. The pathogens themselves and/or their endotoxins and/or exotoxins get into the connective tissue. By their effect and by the effect of the cellular and humoral protection the connective tissue becomes oedematous, its vascular systems expand, and the less acidic exudate and transudate entering the vaginal cavity from the connective tissue also reduce the normal pH of the vagina.

This self-generating process ceases by a wide spectrum biocide treatment, but in the meantime the injured Döderlein flora totally dies out. But the drastic eradication of the weakened protection increases the chance of the relapses. If treatment is carried out with a targeted medicinal product - with a medicine ineffective or less effective for the Döderlein flora - than waiting for the laboratory results delays the beginning of the causal treatment, which results in further destruction of the remaining protection, and the circulus vitiosus continues. Frequent mixed infections make the results of the smear studies and cultivation studies dubious. This is the reason why doctors frequently use ex juvantibus medication or wide spectrum biocide treatment. US5070889 discloses a tampon impregnated with PVP iodine.

According to the patent No. HU218671 the iodine effervescent tablet is a medicine for the preparation of vaginal douche solution, containing polyvinyl-pyrrolidone iodine (hereinunder PVP-iodine), and as effervescent component contains citric acid and sodium hydrogen carbonate for keeping the pH buffered between 3.5-4.3. It washes out the excessive secretion characteristic for the disease. Kills all the local pathogens and the optimal pH of the vagina is kept buffered.

The effective pH-range of the wide spectrum PVP-iodine containing biocide preparations is 2,5-7, optimally between 3-6 (PVP-Iodine, International Specialty Products, 2004.). The disadvantage of such vaginal treating preparations is that they eradicate the Döderlein flora still alive or weakened anyway. After the treatment this further impedes the regeneration of the vaginal bacterial flora.

Therefore a wide spectrum biocide vaginal treating preparation is needed which doesn't hurt the Döderlein flora, or only to a minimal extent.

The effervescent tablet for vaginal douche according to the patent No. HU 215 970 contains lactose and as effervescent component contains citric acid and sodium hydrogen carbonate for keeping the pH buffered between 3.5-4.3.

The effervescent tablet for vaginal douche according to the international patent No. WO2004089278 contains lyophilised mare's milk powder, lactose and as effervescent component contains citric acid and sodium hydrogen carbonate for keeping the pH buffered between 3.5-4.3.

These preparations are suitable for the maintenance of the Döderlein flora, or in case of its minor injury for the regeneration, but cannot be directly combined with biocide substances, because their active ingredients and the biocide substances would reciprocally impair each other's effect, both during storage and during the application of the preparations.

The known biocide vaginal treatment preparations may not be combined with substances degradable or modifiable by biocide substances, for example with prebiotics and/or probiotics necessary for the Döderlein flora. Therefore following the biocide treatment new treatment is necessary for the regeneration of the Döderlein flora.

Therefore there is a need for a biocide vaginal treatment preparation containing biocide active ingredient and which additionally gives the chance for the maintenance and/or regeneration of the Döderlein flora.

Many solutions are known for the direct indication of pH of the vaginal secretion and/or the vaginal wall.

In the patent No. US5217444 adsorbent pad is described containing a pH-indicator substance.

In the international patent No. WO0018345 a tampon is described, the hygroscopic substance of which has a separated indicator-zone, for the indication of the changes in the conditions of the body fluids, and this way for the indication of the changes in their pH.

But these solutions can only detect extended pH-changes, and are unsuitable for the indication for the site of the pH-change.

But for the correct diagnosis a pH indicator is needed, which is able to localize the site of the vaginal pH-deviation.

Medicinal capsule preparations for oral administration are known with enterosolvent coating from the prior art; such a solution is described in the patent No. US6767557.

The present invention relates to the elimination of the disadvantages rooted in the current techniques, and the satisfaction of the currently existing, unmet needs.

Therefore the invention is directed to a biocide preparation, wherein PVP-iodine as an active ingredient which is suitable for colour indication as well, is enclosed in capsules with a wall not dissolving, or dissolving only to a minimal extent in watery environment with a pH range below a specified value - hereinunder "the cutting pH value" - but dissolving in the pH-range over the cutting pH value. The term "cutting pH value" is a pH value of 4,5-6,0.

In this description the expression "capsules" means any size and size-distribution capsules known per se, including the microcapsules.

The PVP-iodine active ingredient is an indicator substance at the same time, which is suitable for colour indication.

The capsules according to the invention can be mixed or embedded into any known vaginal treatment material, i.e. in jellies, suppositories, liquid, concentrates, granule, powder, spermicidal preparations, capsules, tablets, etc., this way preparations containing biocide capsules can be prepared. The invention relates also to these preparations.

The present invention relates also to a tampon, in which, or on the surface of which capsules can be found containing biocide preparation. It must be noted, that the other adsorbent materials containing other sexual-hygienic preparations, which contain the capsules or preparations of the invention also belong to the scope of the present invention.

The following findings led to the realization of the biocide preparation of the invention.

It was recognized that if the wide spectrum biocide substance exerts its effect only where and when the pathogens colonize, then the laboratory tests can generally be omitted, medical treatment can be started earlier, natural protection can be conserved, and there will be less relapses.

If the biocide active ingredient is encapsulated, and the substance of the capsules doesn't dissolve, or dissolves only in negligible amount in the body fluid medium - hereunder for the sake of simplicity in watery medium - in a pH range under the cutting pH value, but dissolves over the cutting pH value, then the vagina, then by selecting the cutting pH value between pH=4,5-6,0 it can be reached that the PVP-iodine doesn't dissolve in the healthy vaginal microenvironment, i.e. in the pH-3,5-4,3 range, and doesn't harm the Döderlein flora. But in more basic micro- and/or macro-environment the PVP-iodine released by the dissolution of the capsules doesn't find Döderlein flora, but it can kill the pathogens.

The load of the capsules contains an indicator substance suitable for colour indication. The capsules dissolving in the medium over the cutting pH value indicate with their colour, that biocide substance was released in the given micro- and/or macro-environment.

The indicator is the same as the active ingredient. The colour of the water solution of the most frequently used biocide active ingredient, the PVP-iodine in the most effective concentration-range, between 0,01-1 % by weight (PVP-Iodine, International Specialty Products, 2004.) is between ochre and brownish-red. As a consequence of this the hue of the biocide preparation according to the invention and/or the liquid used for its rinsing refers also to the incidence of the pathogens. The sensitivity of the indication can be further enhanced, for example with added substances and/or with a special instrument.

The wall of the capsules may also comprise an indicator substance.

In a further preferred embodiment the capsules may contain an additive improving the activity of the biocide substance, adjusting the pH-value, preferably with a high buffer-capacity, in case of vaginal PVP-iodine preparations this means pH=3-4,3. The additive with high buffer capacity can be for example the one according to the patent No. HU 218 671.

According to another finding, if the preparation of the invention is made up of vaginal treating substance and capsules evenly distributed in it, then in the medium characteristic for the vaginal treating substances with pH=3-4,3 the capsules remain stable, this way the vaginal treating preparations can contain additives which otherwise wouldn't be kept in contact with the biocide substances.

This way the biocide active substances and for example the prebiotics and/or probiotics can be used in the same preparation.

In a further preferred embodiment the preparation contains capsules and a substance serving as selective carbon source for the Döderlein flora, and/or a substance originating from the milk of Equidae (horse-like animals) and a buffer system for the generation of the 3,5-4,3 pH-value, for example the buffer system of the patent No. HU 218 671 already referred.

In a further preferred embodiment the preparation contains 10-1000 mg, preferably 300 mg capsules, 0-1000 mg, preferably 700 mg lactose, 0-500 mg, preferably 100 mg lyophilised mare's milk powder, and preferably 2100 mg citric acid and 700 mg sodium hydrogen-carbonate.

The following finding led to the preparation of the tampon of the invention.

If the capsules of the invention are used in tampon and/or on the surface of a tampon, preferably immobilized, then the tampon can fulfil many tasks simultaneously:
- absorbs and stores the body fluids,
- makes treatments with the substances, i.e. with its biocide active ingredient inside it or on its surface
- kills the germs in the absorbed body fluids and/or on the surface in contact with it, and in the meantime doesn't harm the useful, more acidic microflora
- gives information about the presence, quality and quantity of the pathogens with its colour

The colour of the water solution of the PVP-iodine in the most effective concentration-range, between 0,01-1 % by weight (PVP-Iodine, International Specialty Products, 2004.) is between ochre and brownish-red. And if the body fluids are absorbed with a tampon, and a portion of the PVP-iodine containing capsules dissolves, then the colour can be even more variable: if for example the tampon contains cellulose/starch, then it begins with violet. Sensitivity of the indication can be further enhanced with the addition of further special substances. With such an indication new information can be obtained even about the quantity of the pathogens.

According to another finding, if a tampon with cylindrical shape, filling up the total length of the vagina, preferably swelling radially by the effect of the fluid, is used, the surface of the tampon removed after use illustrates as a map all the effects harming the acidic vaginal self protection. If the change of the colour is on the inner, incidentally on the concave end, this indicates that the discharge originates from the orifice of the uterus (wound of the orifice of the uterus), and/or from the portion over it. If the colour changes on the outer end, the substance with abnormal pH enters the vagina from the direction of the labium minus pudendi (nympha) and labium majus pudendi. If an imprint-like colour change can be seen on the abdominal and dorsal superficies, it is generated by a local, definite inflammation or tumorous change. If the colour changes on the inner-lower-back tip of the tampon, this means that an inflammation sitting in the back vault, or, since this is the receptaculum seminis at the same time, the ejaculate may cause it, because its pH is 7,2-7,3.

It was also found, that the reason for the surprisingly late discovery of the vaginal wall cancers is that the metal blade(s) introduced for the investigation cover the front and back walls. After the investigation of the outer orifice of the uterus, as the most important (predilection) site from the aspect of the inflammation and the tumour, the blade is removed without further attention, therefore the early recognition of the vaginal wall cancer frequently gets lost Therefore the vaginal tampon can be a means for the early cancer diagnosis, even the patient using the tampon can call on the attention of the examining doctor.

According to another finding the cylindrical body can be adjusted to the anatomical form of the vagina. The portio vaginalis uteri gets into the seat in inner end of the tampon. The dorsal, labium like stalk of the inner end lies into the back vault. In preferred cases a positioning mark can be at the outer end, which is necessary for the correct insertion, or for the positioned evaluation of the "map" obtained about the pH-deviations. In a further preferred embodiment a supporting surface can be put to the outer end of the cylindrical body, which, after the insertion of the tampon can be pushed with a finger behind the outer surface of the pubis and the symphysis.

In a further preferred embodiment the following dimensions of the vaginal tampon are the most suitable for the dimensions of an average vagina: length of the cylindrical body is 6-15 cm, preferably 11 cm, the diameter of the cylindrical body in dry condition is 0,5-1,5 cm, preferably 1 cm, in wet conditions 1,5-4 cm, preferably 2,5 cm.

In a further preferred embodiment the outer end of the cylindrical body can be put into an applicator, this way the tampon can be inserted positionally.

According to another finding, if the tampon includes a removable indicator coat (an indicator-containing layer), which can be spread out on a flat surface, then the evaluation can be simply carried out, and can be documented even for the doctor.

In a further preferred embodiment the tampon may have a protecting coat removable after the insertion (can be pulled down with a thread after the insertion).

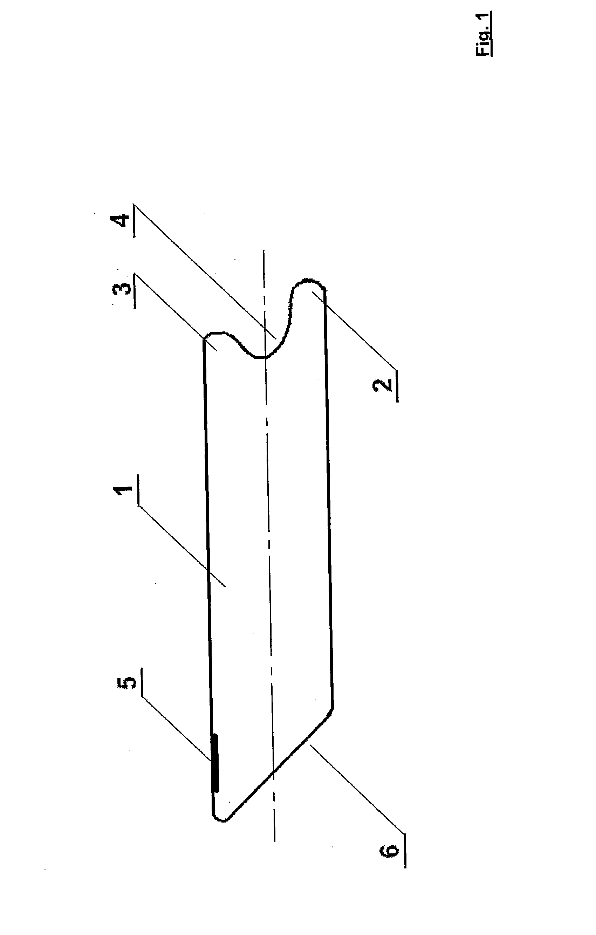

As an example, the possible embodiment of the tampon according to the invention is demonstrated on Figure 1, without limiting the invention to this example.

Figure 1 outlines the structure of the vaginal tampon of the invention.

It is demonstrated on Figure 1 that on the inner end of the of the cylindrical body (1) there is a longer dorsal stalk (2) and a shorter abdominal stalk (3), further in between them there is a seat (4), on the outer end there is a positioning marker (5) and a supporting surface (6). The capsules are uniformly distributed on the whole surface of the vaginal tampon.

After the insertion of the vaginal tampon the positioning marker (5) must direct to the abdomen. The outer end of the inserted vaginal tampon can be pushed with a finger behind the pubis, in such a way, that the supporting surface (6) lies against the pubis and the inner side of the symphysis. This way the position of the vaginal tampon is stable in the vagina, and the dorsal stalk (2) fits into the back vault of the vagina below the portio vaginalis uteri.

By the effect of the fluid absorbed from the surrounding the tampon slowly swells radially.

After 10-60 minutes the vaginal tampon can be pulled out, for example with the threads built into the tampon (not shown on Figure 1).

### Example 1

1 g PVP-iodine and 6 g Eudragit L 100 are dissolved in 23 g 96% ethanol. 100 g lactose is measured in the high-shear Pro-C-epT granulating machine, premix it for 3 minutes, and by dropping the fresh solution with constant speed microparticles are prepared. The maximum dimension of the yellow, finely distributed powder is 160 µm. Following this the particles are coated with 20 ml water suspension of Eudragit L 30D-55 in a MiniGlatt 4 fluid equipment, using 0,10 bar fluid process air and 0,50 bar pulverizing air pressure at 25 °C. The maximum size of the particles obtained this way is 320 µm.

Less active ingredient-dissolution can be observed from the coated particles at pH=4,5 than at pH=6.

## Claims

1. Biocide capsule for using it in a biocide preparation, wherein PVP-iodine as an active ingredient, which is suitable for colour indication as well, is enclosed in capsules with a wall not dissolving, or dissolving only to a minimal extent in watery environment with a pH range below the cutting pH value of 4,5-6,0, but dissolving in the pH-range over the cutting pH value.

2. Capsule of Claim 1, which includes an additive preferably with high buffer capacity, and sets the pH between 3-4,3.

3. Biocide preparation containing a biocide capsule of Claims 1 or 2, which includes a substance for the treatment of the vagina, and capsules evenly distributed in the substance for the treatment of the vagina.

4. Preparation of Claim 3, which includes one or more prebiotics and/or probiotics.

5. Preparation of Claims 3 or 4, wherein the vaginal treatment substance includes a selective carbon source for the Döderlein flora and/or a substance from the milk of Equidae (horse-like animals) and a buffer system suitable for the generation of the 3,5-4,3 pH-value.

6. Preparation of Claim 5, which includes 10-1000 mg, preferably 300 mg capsules, 0-1000 mg, preferably 700 mg lactose, 0-500 mg, preferably 100 mg lyophilised mare's milk powder, and preferably 2100 mg citric acid and 700 mg sodium hydrogen-carbonate.

7. Tampon, having inside or on the surface capsules, preferably immobilized, of any of Claims 1 or 2, or biocide preparation of any of Claims 3-6.

8. Tampon of Claim 7, which is a hygroscopic cylindrical body (1), preferably swelling radially for fluids, having at the inner end a longer dorsal stalk (2), insertable until the back vault of the vagina and an abdominal stalk (3) shorter than the dorsal stalk, and in between them there is a seat (4) accepting the portio vaginalis uteri.

9. Tampon of Claim 8, which has at the outer end of the cylindrical body (1) a positioning marker (5) and/or an inclined supporting surface (6) which can be pushed with a finger behind the pubis and the inner side of the symphysis.

10. Tampon of any of Claims 7-9, having an indicator-containing coat, after use preferably removable and spreadable on a flat surface.

11. Tampon of any of Claims 7-10, having a coat, preferably removable after insertion.

12. Tampon of any of Claims 7-11, wherein the outer end of the cylindrical body (1) can be put into an applicator, to provide a preferably positional insertion of the tampon into the body cavity.

13. Tampon of any of Claims 7-12, wherein the length of the cylindrical body (1) is 6-15 cm, preferably 11 cm, the diameter of the cylindrical body (1) in dry conditions is 0,5-1,5 cm, preferably 1 cm, in wet conditions 1,5-4 cm, preferably 2,5 cm

## Patentansprüche

1. Biozidkapsel zur Verwendung in einer Biozidzubereitung, wobei PVP-Iod als Wirkstoff, das auch zur Farbindikation geeignet ist, in Kapseln eingeschlossen ist, mit einer Wand, die sich in wässriger Umgebung mit einem pH-Bereich unter dem trennenden pH-Wert von 4,5-6,0 nicht auflöst oder nur bis zu einem minimalen Grad auflöst, sich aber im pH-Bereich über dem trennenden pH-Wert auflöst.

2. Kapsel nach Anspruch 1, die einen Zusatz, vorzugsweise mit hoher Pufferkapazität, enthält und den pH zwischen 3 und 4,3 einstellt.

3. Biozidzubereitung, die eine Biozidkapsel nach Anspruch 1 oder 2 enthält, die eine Substanz zur Behandlung der Vagina und Kapseln, die in der Substanz gleichmäßig zur Behandlung der Vagina verteilt sind, enthält.

4. Zubereitung nach Anspruch 3, die ein Präbiotikum oder mehrere Präbiotika und/oder Probiotika enthält.

5. Zubereitung nach Anspruch 3 oder 4, wobei die Vaginalbehandlungssubstanz eine selektive Kohlenstoffquelle für die Döderlein-Flora und/oder eine Substanz aus der Milch von Equidae (pferdeartigen Tieren) und ein Puffersystern enthält, das für die Erzeugung des pH-Wertes von 3,5 bis 4,3 geeignet ist.

6. Zubereitung nach Anspruch 5, die 10-1000 mg, vorzugsweise 300 mg Kapseln, 0-1000 mg, vorzugsweise 700 mg Lactose, 0-500 mg, vorzugsweise 100 mg lyophilisiertes Stutenmilchpulver und vorzugsweise 2100 mg Zitronensäure und 700 mg Natriumhydrogencarbonat enthält.

7. Tampon, der im Inneren oder an der Oberfläche Kapseln nach Anspruch 1 oder 2, vorzugsweise immobilisiert, oder eine Biozidzubereitung nach einem der Ansprüche 3 bis 6 aufweist.

8. Tampon nach Anspruch 7, der ein hygroskopischer zylindrischer Körper (1) ist, der vorzugsweise radial für Fluida quillt, der am inneren Ende einen längeren dorsalen Schaft (2), der bis zum hinteren Gewölbe der Vagina einsetzbar ist, und einen abdominalen Schaft (3), der kürzer als der dorsale Schaft ist, aufweist, und wobei sich zwischen ihnen ein Sitz (4) befindet, der die *Portio vaginalis uteri* ("Scheidenteil der Gebärmutter") aufnimmt.

9. Tampon nach Anspruch 8, der am äußeren Ende des zylindrischen Körpers (1) einen Positionierungsmarker (5) und/oder eine geneigte Stützfläche (6) hat, die mit einem Finger hinter das Schambein und die Innenseite der Symphyse geschoben werden kann.

10. Tampon nach einem der Ansprüche 7 bis 9 mit einer indikatorhaltigen Beschichtung, der nach Verwendung entfernbar und auf einer flachen Oberfläche ausbreitbar ist.

11. Tampon nach einem der Ansprüche 7 bis 10 mit einer Beschichtung, der vorzugsweise nach dem Einsetzen entfernbar ist.

12. Tampon nach einem der Ansprüche 7 bis 11, wobei das äußere Ende des zylindrischen Körpers (1) in einen Applikator eingebracht werden kann, um für ein vorzugsweise positionelles Einsetzen des Tampons in die Körperhöhle zu sorgen.

13. Tampon nach einem der Ansprüche 7 bis 12, wobei die Länge des zylindrischen Körpers (1) 6 bis 15 cm, vorzugsweise 11 cm ist, der Durchmesser des zylindrischen Körpers (1) unter trockenen Bedingungen 0,5 bis 1,5 cm, vorzugsweise 1 cm, unter nassen Bedingungen 1,5 bis 4 cm, vorzugsweise 2,5 cm ist.

## Revendications

1. Gélule de biocide pour être utilisée dans une préparation biocide, dans laquelle le PVP-iode en tant que principe actif et pouvant convenir également comme indicateur de couleur, est enfermé dans des gélules à paroi non dissolvante, ou ne se dissolvant que dans une proportion minimale en milieu aqueux dans une plage de pH inférieure à la valeur du pH de coupe allant de 4,5 à 6, mais se dissolvant dans une plage de pH supérieure à la valeur du pH de coupe.

2. Gélule selon la revendication 1, qui comprend un additif de préférence à fort pouvoir tampon et qui établit le pH entre 3 - 4,3.

3. Préparation de biocide contenant une gélule de biocide selon les revendications 1 ou 2, qui comprend une substance pour le traitement du vagin, et des gélules uniformément distribuées dans la substance pour le traitement du vagin.

4. Préparation selon la revendication 3, qui comprend un ou plusieurs prébiotiques et/ou probiotiques.

5. Préparation selon les revendications 3 ou 4, dans laquelle la substance de traitement vaginale comprend une source de carbone sélective pour la flore de Doderlein et/ou une substance à partir du lait d'équidés (animaux du type chevaux) et un système de tampon approprié pour la génération d'une valeur de pH de 3,5-4,3.

6. Préparation selon la revendication 5, qui comprend de 10 à 1000 mg, de préférence 300 mg de gélules, de 0 à 1000 mg, de préférence 700 mg de lactose, de 0 à 500 mg, de préférence 100 mg de poudre de lait de jument lyophilisé, et de préférence 2100mg d'acide citrique et 700 mg d'hydrogénocarbonate de sodium.

7. Tampon hygiénique comprenant, à l'intérieur ou sur la surface, des gélules, de préférence immobilisées, selon la revendication 1 ou 2, ou la préparation biocide selon l'une quelconque des revendications 3-6.

8. Tampon selon la revendication 7, qui consiste en un corps cylindrique hygroscopique (1), de préférence gonflant radialement pour fluides, comprenant à l'extrémité intérieure une tige dorsale plus longue (2), pouvant être insérée jusqu'au fond du vagin et une tige abdominale (3) plus courte que la tige dorsale, et entre eux, est disposé un siège (4) acceptant la portion sus-vaginale du col de l'utérus.

9. Tampon selon la revendication 8, qui possède à l'extrémité extérieure du corps cylindrique (1) un marqueur de positionnement (5) et/ou une surface d'appui inclinée (6) qui peut être poussée avec un doigt derrière le pubis et le côté intérieur de la symphyse pubienne.

10. Tampon selon l'une quelconque des revendications 7 à 9, comprenant une couche contenant des indicateurs, de préférence amovible après usage et susceptible d'être étalée sur une surface plane.

11. Tampon selon l'une quelconque des revendications 7 à 10, comprenant une couche, de préférence amovible après insertion.

12. Tampon selon l'une quelconque des revendications 7 à 11, dans laquelle l'extrémité extérieure du corps cylindrique (1) peut être mise dans un applicateur, pour obtenir une insertion préférable de la position du tampon dans la cavité corporelle.

13. Tampon selon l'une quelconque des revendications 7 à 12, dans laquelle la longueur du corps cylindrique (1) est de 6 à 15 cm, de préférence de 11 cm, le diamètre du corps cylindrique (1) dans des conditions sèches est de 0,5 à 1,5 cm , de préférence de 1cm, et dans des conditions humides, de 1,5 à 4 cm, de préférence de 2,5 cm.
